# EUROPÄISCHE PATENTSCHRIFT

(11) **EP 3 054 902 B1**
(45) Veröffentlichungstag und Bekanntmachung des Hinweises auf die Patenterteilung: **06.12.2017**
(21) Anmeldenummer: 14795554.6
(22) Anmeldetag: 10.10.2014
(51) Int. Cl.: A61F 2/60, A61F 2/64, A61F 2/68, A61F 2/74

(54) **ORTHOPÄDIETECHNISCHE DÄMPFEREINRICHTUNG**
ORTHOPEDIC DAMPER DEVICE
DISPOSITIF D'AMORTISSEMENT ORTHOPEDIQUE

(30) Priorität: 10.10.2013 DE 102013016800
(43) Veröffentlichungstag der Anmeldung: 17.08.2016
(73) Patentinhaber: Otto Bock HealthCare GmbH, 37115 Duderstadt (DE)
(72) Erfinder: SCHUH, Andreas, 37115 Duderstadt (DE); KIMMIG, Clemens, 77728 Oppenau (DE)
(74) Vertreter: Stornebel, Kai
(86) Internationale Anmeldenummer: PCT/EP2014/002744
(87) Internationale Veröffentlichungsnummer: WO 2015/051918

(56) Entgegenhaltungen:
- WO-A1-00/74610
- DE-A1-102010 031 723
- DE-U1-202004 008 014
- DE-U1-202009 016 261
- GB-A- 982 527

## Beschreibung

Die Erfindung betrifft eine orthopädietechnische Dämpfereinrichtung mit einem beweglich gelagerten Kolben und zumindest einer eine Wandung aufweisende Kammer, in der ein komprimierbares Medium durch Verlagerung des Kolbens in einer ersten Richtung verdichtet und durch Verlagerung des Kolbens in eine entgegensetzte zweite Richtung entspannt wird.

Die orthopädietechnische Dämpfereinrichtung ist insbesondere bei Orthesengelenken oder Prothesenkniegelenken einsetzbar, kann aber auch grundsätzlich zur Verbesserung des Verhaltens pneumatischer Dämpfer eingesetzt werden.

Beim Einsatz von orthopädietechnischen Dämpfereinrichtungen, die ein komprimierbares Medium verwenden, wird das Medium in einer Kompressionskammer verdichtet, indem ein Kolben das Kammervolumen verringert. Der Kolben kann sowohl als linear verschiebbarer Kolben als auch als Rotationskolben ausgebildet sein. Wird die auf den Kolben ausgeübte Kraft verringert, beispielsweise bei einer Bewegungsumkehr, entspannt sich das komprimierte Medium, beispielsweise Luft, und übt eine Rückstellkraft aus. Bei prothetischen oder orthetischen Gelenken kann dies zu einem Katapult-Effekt einer Gelenkkomponente führen, beispielsweise bei einer Unterschenkelkomponente, was insbesondere bei hohen Bewegungsgeschwindigkeiten nachteilig ist.

Die WO 00/74610 A1 betrifft eine Vorrichtung zur Höhensteuerung einer Beinprothese mit einem Zylinder, einem darin geführten Kolben und einer Kolbenstange, wobei die Kolbenstange aus einem Ende des Zylinders ausgeführt ist. Der Zylinder und die Kolbenstange sind jeweils mit einem von zwei Verbindungsteilen verbunden, die wiederum mit Teilen der Beinprothese verbunden sind. Ein Kanal zur Leitung eines Mediums von einer Seite des Kolbens zur anderen durch ein Ventil hindurch ist vorhanden. Das Ventil ist durch den Prothesennutzer betätigbar. In dem Zylinder ist ein pneumatisches Medium enthalten. Nach dem Öffnen des Ventils kann das Druckmedium durch den Kanal fließen.

Aufgabe der vorliegenden Erfindung ist es, eine orthopädietechnische Dämpfereinrichtung bereitzustellen, mit der eine bessere Anpassbarkeit des Expansionsverhaltens und insbesondere des Extensionsverhaltens erreicht werden kann.

Erfindungsgemäß wird diese Aufgabe durch eine orthopädietechnische Dämpfereinrichtung mit den Merkmalen des Hauptanspruches gelöst. Vorteilhafte Ausgestaltungen und Weiterbildungen der Erfindung sind in den Unteransprüchen, der Beschreibung und den Figuren offenbart.

Die erfindungsgemäße orthopädietechnische Dämpfereinrichtung mit einem beweglichen gelagerten Kolben und zumindest einer eine Wandung aufweisende Kammer, in der ein komprimierbares Medium durch Verlagerung des Kolbens in einer Richtung verdichtet und durch Verlagerung des Kolbens in eine entgegensetzte zweite Richtung entspannt wird, sieht vor, dass die Kammer mit einem Auslasskanal verbunden ist, dass dem Auslasskanal ein Verschlusselement zugeordnet ist, dass das Verschlusselement beweglich an einem Träger gelagert, dass der Träger mit dem Kolben gekoppelt oder als Kolben ausgebildet ist und dass das Verschlusselement mit einem relativ zu dem Träger verlagerbaren Kontaktbereich gekoppelt ist. Durch die-se Ausgestaltung ist es möglich, eine positionsunabhängige und richtungsgesteuerte Entlüftung der Kammer zu bewirken und den Auslasskanal in dem Moment freizugeben, wenn keine Kompression des Mediums mehr erfolgt und eine Entspannung des Mediums erfolgen sollte. Durch die positionsunabhängige und richtungsgesteuerte Entlüftung zum Umkehrzeitpunkt, beispielsweise von einer Flexion zu einer Extension, ist es möglich, einerseits den Dämpfungs- und Polstereffekt durch die Kompression des Mediums in der Kammer auszunutzen und andererseits zu verhindern, dass durch den Druck auf den Kolben eine Rückstellbewegung forciert wird bzw. die Rückstelltendenz vermindert wird. Der Träger dient in bevorzugten Ausgestaltungen als Betätigungselement für das Verschlusselement. Wird nachfolgend von einer Dämpfereinrichtung gesprochen, handelt es sich um eine orthopädietechnische Dämpfereinrichtung, insbesondere für Orthesengelenke oder Prothesengelenke, insbesondere für Kniegelenke. Der Träger kann als Kolben, Kolbenstange, Zylinderwandung, Kammerwandung in einem Sperrelement oder als Sperrelement ausgebildet sein.

Eine Weiterbildung sieht vor, dass der Kontaktbereich, mit dem das Verschlusselement gekoppelt ist, die Wandung der Kammer oder die Wandung eines Sperrelementes ist, die in der Kammer oder in einem kraft- oder bewegungstechnisch gekoppelten Bereich angeordnet ist.

Der Kolben kann als Drehkolben oder zylindrischer Kolben mit einer linearen, reziproken Bewegungsbahn ausgebildet sein.

Der Auslasskanal kann in die Umgebungsatmosphäre oder in eine zweite Kammer münden. Bei der Ausgestaltung mit zwei Kammern ist es möglich, dass der Auslasskanal in die entgegengesetzt wirkende Kammer mündet, bei einer Gelenkeinrichtung wären dies die Flexionskammer und die Extensionskammer.

In oder an dem Auslasskanal kann ein Schalldämpfer angeordnet sein, um das Geräusch des entweichenden Mediums zu verringern. Dies ist insbesondere bei Orthesen oder Prothesen vorteilhaft, um das Ausstoßgeräusch für den Nutzer der Dämpfereinrichtung und die Umgebung zu minimieren.

Das Verschlusselement kann reibend und gleitend an dem Kontaktbereich gelagert sein, so dass durch eine Reibkraft die Umschaltung des Verschlusselementes von dem Verschlusszustand zum geöffneten Zustand erfolgen kann.

Der Auslasskanal kann durch den Kolben selbst geführt sein, wodurch sich eine einfache konstruktive Lösung ergibt.

Das Verschlusselement kann gegen den Kontaktbereich elastisch vorgespannt sein, wodurch es möglich wird, dass eine gleichbleibende Kontaktkraft im Kontaktbereich vorliegt. Das Verschlusselement ist somit selbst nachstellend ausgestaltet, wobei ein Spannelement vorgesehen sein kann, über das die Reibungskraft des Verschlusselementes eingestellt werden kann.

Das Verschlusselement kann als Kolbenring ausgebildet und in einer Kolbenringnut gelagert sein, so dass in der einen Richtung die Kompression des komprimierbaren Mediums, insbesondere Luft, gewährleistet wird, in der anderen Bewegungsrichtung durch Ausbildung eines ausreichenden Spiels und eines Auslasskanals die komprimierte Luft bei Bewegungsumkehr des Kolbens durch den Auslasskanal entweichen kann.

Das Verschlusselement kann auch an einer Kolbenstange und in einem die Kolbenstange umgebenden Sperrelement gelagert sein. Das Sperrelement kann innerhalb des Zylinders angeordnet sein, in dem der Kolben geführt ist. Das Sperrelement kann die jeweiligen Kammern abgrenzen und fest in dem Gehäuse oder dem Zylinder angeordnet sein. Das Sperrelement kann auch die Funktion des Trägers übernehmen.

Das Verschlusselement kann im verschließenden Zustand an einer den Auslasskanal umgebenden Dichtung anliegen, wobei die Dichtung als O-Ring ausgebildet sein kann, der um den Auslasskanal herum angeordnet ist und leicht über diesen hinaussteht, so dass durch die Anlage des Verschlusselementes an die Dichtung ein luftdichter Verschluss des Auslasskanals gegeben ist.

Eine zweite Kammer kann auf der der ersten Kammer gegenüberliegenden Seite des Kolbens angeordnet sein, in der das komprimierbare Medium verdichtet wird. Bei einer solchen Ausgestaltung liegt ein doppeltwirkender Kolben vor, der zur Dämpfung sowohl der Extensions- als auch Flexionsbewegung oder der Bewegung in einer ersten und in einer zweiten Richtung eingesetzt werden kann. Je nach Orientierung des Auslasskanals und des Verschlusselementes kann jeweils die Rückstellkraft durch das expandierende Medium eingestellt werden. Es ist auch möglich, dass beide Kammern mit einem Auslasskanal und einem Verschlusselement zur richtungsgesteuerten Entlüftung der jeweiligen Kammer gekoppelt sind.

Das Sperrelement kann in einem die Kammern ausbildenden Gehäuse ortsfest gelagert sein, beispielsweise ist es möglich, dass das Sperrelement die erste Kammer von einem doppeltwirkenden Dämpfungszylinder abtrennt, so dass insgesamt zumindest drei Kammern ausgebildet sind, von denen zwei jenseits des Sperrelementes angeordnet sind.

Das Verschlusselement kann über ein Schaltelement mit dem Kolben gekoppelt sein, so dass keine unmittelbare Zuordnung des Verschlusselementes mit dem Kolben oder dem Kontaktbereich notwendig ist.

Das Verschlusselement und/oder der Auslasskanal können einstellbar gelagert oder ausgebildet sein, um den Grad der Maximalentlüftung einstellen zu können. Durch die einstellbare Ausgestaltung kann das Verschlusselement auch vollständig deaktiviert werden, falls eine schnelle Entlüftung nicht gewünscht wird.

Eine Weiterbildung der Erfindung sieht vor, dass die Kammer in Richtung auf eine Endstellung des Kolbens konisch verjüngt ausgebildet ist. Durch die Verjüngung wird erreicht, dass sich im Bereich der Endstellung der Abstand zwischen dem Verschlusselement und der Zylinderwandung oder der Kammer verringert, so dass es zu einem verbesserten Kontakt zwischen dem Verschlusselement und der Kammerwandung kommt, wenn der Kontaktbereich ein Teil der Kammerwandung ist. Dabei ist eine vergleichsweise geringe Konizität ausreichend, um eine Verbesserung des Ansprechverhaltens des Verschlusselementes zu erzielen. Die Ausgestaltung einer sich konisch verjüngenden Kammer kann durch Rollieren erfolgen, wodurch zudem eine Glättung und Verfestigung der Oberfläche der Kammer erreicht wird. Die Konizität kann über die gesamte Länge der Kammer oder auch nur über einen gewissen Teil vorgesehen sein. Der Konuswinkel kann zwischen 0,2 ° und 1 ° betragen, vorzugsweise 0,5 °. Alternativ zu einer konischen Verjüngung einer Kammer oder einer Zylinderbohrung im Endbereich der Kolbenbewegung ist es auch möglich und vorgesehen, dass bei einer Ausgestaltung des Trägers als Kolbenstange diese im Bereich der Endstellung eine Erweiterung, also Durchmesservergrößerung aufweist. Liegt das Verschlusselement oder das Schaltelement von außen an der Kolbenstange an, erhöht sich im Bereich der Erweiterung der Druck und dadurch die Reibkraft, was zu einer sicheren Schaltausführung führt. Auch die Erweiterung kann konstant über den gesamten Relativverschiebeweg zwischen Träger und Schalt- oder Verschlusselement sich verändern oder nur abschnittsweise, so dass eine Vergrößerung nur in bestimmten Abschnitten vorliegt.

Der Träger kann ortsfest innerhalb der Kammer angeordnet sein und dadurch eine Kammerunterteilung bewirken. Die Kolbenstange kann durch den Träger bzw. eine Kammerabgrenzung hindurchgehen.

Vorteilhafterweise ist die zweite Kammer, die mit der ersten Kammer in strömungstechnischer Verbindung steht, als offene Kammer ausgebildet und weist einen gegenüber der ersten Kammer geringeren Innendruck auf, wenn eine Kompression der ersten Kammer erfolgt. Ein Gegendruck, der über den Umgebungsdruck hinausgeht, wird in der zweiten Kammer nicht aufgebaut. Dadurch ist es möglich, dass über die Dämpfereinrichtung eine ungewünschte Federwirkung, am Ende beispielsweise einer Flexionsbewegung, aufgrund der eingeschlossenen Luftvolumina auftritt. Vielmehr wird bei einer geringfügigen Rückbewegung, also Extension, die Auslassöffnung des Auslasskanals durch das Verschlusselement geöffnet, so dass der in der ersten Kammer herrschende Überdruck abgebaut und ein Rückfedereffekt vermieden wird. In der zweiten Kammer herrscht bevorzugt Umgebungsdruck.

Nachfolgend werden Ausführungsbeispiele der Erfindung anhand der beigefügten Figuren näher erläutert. Es zeigen:
- Figur 1: eine schematische Schnittdarstellung einer Dämpfereinrichtung;
- Figur 2: eine Detailansicht;
- Figur 3: eine perspektivische Darstellung eines Kolbens;
- Figur 4: eine teiltransparente Darstellung eines Kolbens;
- Figur 5: eine Darstellung eines Kolbens mit herausgehobenen Luftkanälen;
- Figur 6: eine Schnittdarstellung eines Kolbens;
- Figur 7: eine perspektivische Schnittdarstellung einer ersten Ausführungsform;
- Figur 8: eine Detaildarstellung eines Verschlusselementes in geschlossener Position;
- Figur 9: eine Detaildarstellung gemäß Figur 8 in offener Position;
- Figur 10: eine Gesamtschnittdarstellung eines Kolbens;
- Figur 11: eine Schnittdarstellung einer Variante der Erfindung;
- Figur 12: eine Detailansicht eines Kolbens gemäß Figur 11 mit geöffnetem Verschlusselement;
- Figur 13: eine Darstellung gemäß Figur 12 mit geschlossenem Verschlusselement;
- Figur 14: eine perspektivische Darstellung eines Kolbens gemäß Figuren 12 oder 13; sowie
- Figur 15 -: eine Variante der Figur 11.

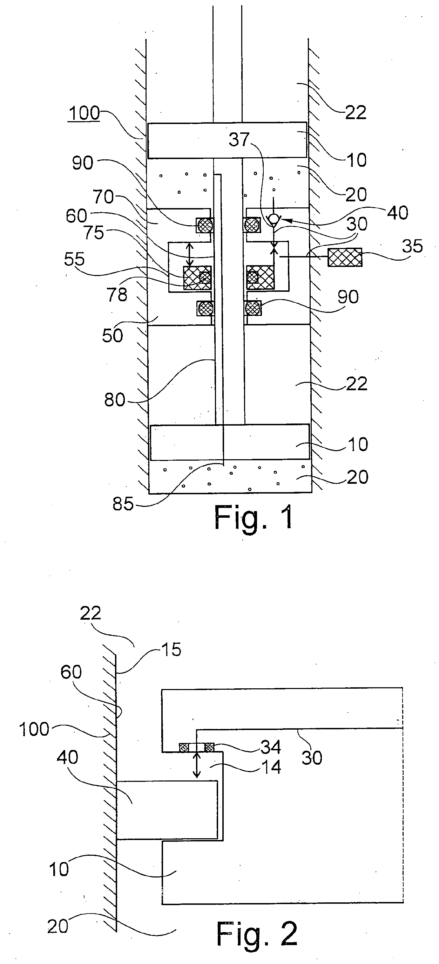

In der Figur 1 ist in einer schematischen Schnittdarstellung eine Dämpfereinrichtung mit einem Gehäuse 100 gezeigt, in dem zwei Kolben 10 längsbeweglich angeordnet sind. Die Kolben 10 sind durch eine Kolbenstange 80 miteinander verbunden und bilden zusammen mit dem Gehäuse und einem Träger 50 zwei erste Kammern 20 aus, in denen das darin befindliche Medium, beispielsweise Luft, bei einer nach unten gerichteten Bewegung komprimiert wird. Zwischen dem unteren Kolben 10 und der Unterkante des Trägers 50 ist eine zweite Kammer 22 ausgebildet, die ebenfalls mit dem komprimierbaren Medium befüllt ist und wobei bei einer gegenläufigen Bewegung, also bei einer Bewegung des unteren Kolbens 10 in Richtung auf den Träger 50, das Medium komprimiert wird. Die Dämpfereinrichtung kann beispielsweise in Prothesen oder Orthesen eingesetzt werden, beispielsweise in Prothesenkniegelenke oder Orthesenkniegelenke, wobei die bevorzugte Einbausituation aufgrund der auftretenden hohen Kräfte vorsieht, dass die beiden ersten Kammern 20 einer Flexion bei einem Kniegelenk entgegenwirken, während die zweite Kammer 22 einer Extension entgegenwirkt. Dementsprechend kann von den ersten Kammern 20 als Flexionskammer und von der zweiten Kammer 22 als Extensionskammer gesprochen werden.

Die Kolbenstange 80 ist durch den Träger 50 hindurchgeführt, Dichtungen 90 liegen an der Kolbenstange 80 an, so dass ein wesentlicher gasdichter Abschluss vorliegt und kein Medium von der oberen Flexionskammer 20 in die Extensionskammer 22 durch den Träger 50 hindurch einströmen kann. An dem Kolben 10 sind nicht dargestellte Dichtungen angeordnet, die ebenfalls ein Vorbeiströmen des komprimierten Mediums an der Außenwand entlang verhindern. Ein ungewollter Gasdurchtritt von der Flexionskammer 20 in die Extensionskammer 22 oder in die Umgebung ist somit nicht möglich. Oberhalb des oberen Kolbens 10 kann, sofern ein obenseitiger Abschluss erfolgt, eine zweite Extensionskammer 22 ausgebildet werden.

Der untere Kolben 10 und die Kolbenstange 80 sind mit einem Verbindungskanal 85 versehen, so dass die untere Flexionskammer 20 und die obere Flexionskammer 20 strömungstechnisch miteinander in Verbindung stehen. Innerhalb des Trägers 50 ist eine Aktuatorkammer 55 ausgebildet, die als Ringraum ausgestaltet sein kann, in der ein Schaltelement 75 angeordnet ist. Das Schaltelement 75 ist über ein Gleit- und Reibelement 78, das beispielsweise als O-Ring ausgebildet sein kann, reibend an der Kolbenstange 80 gelagert. Die Aktuatorkammer 55 ist so dimensioniert, dass das Schaltelement 75 über einen gewissen Schaltweg hinweg verlagert werden kann. Der Schaltweg ist durch den Doppelpfeil angedeutet.

Innerhalb des Trägers 50 ist ein Verschlusselement 40 in Gestalt eines Schaltventils angeordnet, das über einen Auslasskanal 30 mit der Aktuatorkammer 55 verbunden ist. Von der Flexionskammer 20 kann Luft bei einem geöffneten Verschlusselement 40 in die Aktuatorkammer 55 und von dort über einen weiteren Auslass-kanal 30 durch einen Schalldämpfer 35 in die Umgebung entweichen. Die Dämpfereinrichtung gemäß Figur 1 komprimiert bei einer Abwärtsbewegung der Kolben 10 durch die Verringerung der Kammervolumina der Flexionskammern 20 das komprimierbare Medium, wobei sich aufgrund des Verbindungskanals 85 ein Druckausgleich zwischen beiden Flexionskammern 20 einstellt. Aufgrund der Orientierung des Verschlusselementes 40 in Gestalt eines Rückschlagventils kann keine Luft aus der oberen Flexionskammer 20 durch den Auslasskanal 30 entweichen. Aufgrund der Reibung zwischen dem Schaltelement 75 und der Kolbenstange 80, beispielsweise aufgrund der O-Ringdichtung 78 wird das Schaltelement 75 an die untere Begrenzung der Aktuatorkammer 55 verschoben, wobei eine mögliche Kopplung des Schaltelementes 75 mit dem Verschlusselement 40 außer Eingriff gebracht wird. Aufgrund der hohen Kompression innerhalb der Flexionskammern 20 neigen bei einer Bewegungsumkehr des Gelenkes, also bei einer Extension, die beteiligten Gelenkkomponenten zu einem nach vorne Schnellen oder einer sehr schnellen Extension und einer Bewegungsunterstützung, was zu einem ungewollten Bewegungsablauf führen kann. Bei einer Bewegungsumkehr der Kolben 10 und damit auch der Kolbenstange 80 wird das Schaltelement 75 mit in Richtung der Extensionsbewegung verlagert, was durch den Doppelpfeil angedeutet wird. Über ein Betätigungselement, beispielsweise einen Bolzen, wird bei einer Aufwärtsbewegung des Schaltelementes 75 das Rückschlagventil oder Verschlusselement 40 geöffnet, so dass das komprimierbare Medium innerhalb der Flexions-kammern 20 aus den Auslasskanälen 30 in die Umgebung durch den Schalldämpfer 35 entweicht. Das Verschlusselement 40 wird so lange offen gehalten, wie das Schaltelement 75 in der Schaltposition, beispielsweise am oberen Ende der Aktuatorkammer 55 befindlich ist. Bei einer erneuten Bewegungsumkehr, also einer zusätzlichen Flexion, wird das Schaltelement 75 außer Eingriff mit dem Verschlusselement 40 gebracht und die Kammervolumina innerhalb der Flexionskammern 20 sind wieder geschlossen, so dass eine effektive Flexionsdämpfung aufgrund der pneumatischen Kompression erreicht werden kann.

Das Schaltelement 75 ist somit über einen Kontaktbereich 60, der im Wesentlichen der Länge des Hubes der Kolbenstange 80 entspricht, mit dem Verschlusselement 40 gekoppelt.

Das Schaltelement 75 drückt in dem in Figur 1 gezeigten Ausführungsbeispiel gegen einen Stößel 37, der in Figur 1 von oben in die Aktuatorkammer 55 hineinragt.

Der Stößel 37 ist beweglich gelagert und wird beim Anheben des Schaltelementes 75 in den Träger 50 verschoben und öffnet auf diese Weise das Verschlusselement 40.

In der Figur 2 ist eine Variante der Erfindung dargestellt, bei der eine Detailansicht der Dämpfereinrichtung gezeigt ist. Der Kolben 10 ist innerhalb des Gehäuses 100 entlang einer Zylinderwandung 15 hin- und her beweglich gelagert. Am Kolbenumfang ist eine Kolbenringnut 14 ausgebildet, in der ein Kolbenring als Verschlusselement 40 angeordnet ist. Die Kolbenringnut 14 weist ein Übermaß zu der Breite des Kolbenringes auf, so dass der Kolbenring 40 als Verschlusselement ein leichtes Spiel in Verlagerungsrichtung des Kolbens 10 aufweist. Die Möglichkeit der Relativbewegung ist durch den Doppelpfeil angedeutet. Zwischen dem Außenumfang des Kolbens 10 und der Wandung 15 des Zylinders ist ein geringes Spiel vorhanden, das in der Figur 2 übertrieben dargestellt ist. Der Kolbenring 40 weitet sich radial nach außen auf und liegt beweglich an der Wandung 15 an. Die Wandung 15 bildet gleichzeitig den Kontaktbereich zwischen dem Verschlusselement 40 und dem Kolben 10, der wiederum als Träger für das Verschlusselement 40 ausgebildet ist. Innerhalb des Kolbens 10 ist ein Auslasskanal 30 eingearbeitet, die im Wesentlichen senkrecht zu der Wandung 14 des Zylinders orientiert ist. Um die Mündung des Auslasskanals 30 im Bereich der senkrecht zu der Wandung 15 orientierten Nutwand ist eine Dichtung 34 angeordnet, beispielsweise in Gestalt eines O-Ringes. Bei einer Aufbewegung des Kolbens 10, wird der Kolbenring 40 innerhalb der Kolbenringnut 14 aufgrund der Reibung in Richtung auf die der Dichtung 34 gegenüberliegende Seitenwand gedrückt, so dass ein Spalt zwischen der Kammer 20 und dem Auslasskanal 30 geöffnet wird. Ein komprimierbares Medium kann zwischen der Wandung 15, dem Kolbenaußendurchmesser 10 und dem Spalt zwischen dem Kolbenring 40 und der Nut 14 hindurch in den Auslasskanal 30 gelangen und von dort aus, beispielsweise über eine Kolbenstange, in die Umgebung entweichen. Bei einer Bewegungsumkehr, wenn eine Flexionsbewegung eingeleitet wird, wird der Kolben 10 nach unten bewegt, aufgrund der Reibung des Kolbenringes 40 wird dieses als Verschlusselement wirkende Bauteil gegen die Dichtung 34 gedrückt, so dass der Auslasskanal 30 verschlossen wird. Während der Flexionsbewegung bleibt die Flexionskammer 20 verschlossen, so dass das Medium komprimiert wird. Aufgrund der Konstruktion ist es möglich, eine belastungsunabhängige, richtungsgesteuerte Entlüftung der Flexionskammer 20 oder Flexions-kammern 20 zu erreichen.

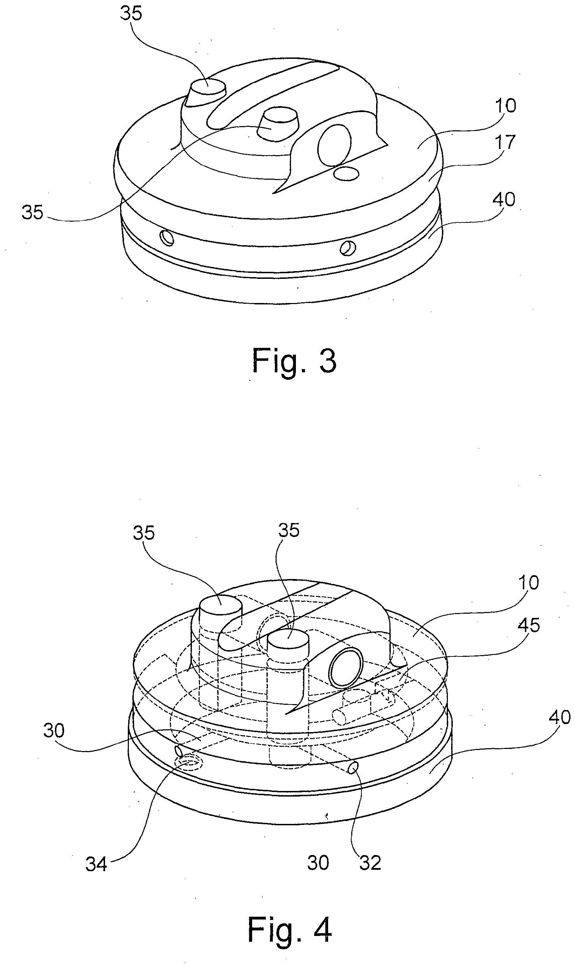

In der Figur 3 ist ein Kolben 10 mit dem Kolbenring 40 am Außenumfang dargestellt. Der Kolbenring 40 ist in der Kolbenringnut 14 eingelegt, oberhalb des Kolbenrings 40 ist ein weiterer Dichtring 17 angeordnet, um die Flexionskammer gegen ungewollte Entlüftung abzudichten. Im Bereich der Kolbenstangenaufnahme sind zwei Schalldämpfer 35 für Auslasskanäle vorgesehen.

In der Figur 4 ist der Kolben 10 in einer teiltransparenten Darstellung gezeigt. Der obere Dichtring 17 ist entfernt, der Kolbenring 40 als Verschlusselement ist in der Nut eingelegt. Es ist zu erkennen, dass die Auslasskanäle 30 über Querbohrungen, die radial nach innen geführt sind, innerhalb des Kolbens 10 ausgebildet sind. Die Auslasskanäle 30 sind durch Stopfen 32 verschlossen. Innerhalb der Nut 14 ist die Dichtung 34 an der oberen Seitenwand der Kolbenringnut zu erkennen. Über die Auslasskanäle 30 gelangt die Luft aus der Flexionskammer durch die Schalldämpfer 35 in die Umgebungsatmosphäre. Der Dichtung 34 gegenüberliegend ist ein Spannelement 45 für das Verschlusselement 40 angeordnet, mit dem es möglich ist, eine radiale Vorspannung zu erreichen und aufrechtzuerhalten, so dass eine konstante Anpresskraft über einen langen Zeitraum gewährleistet wird.

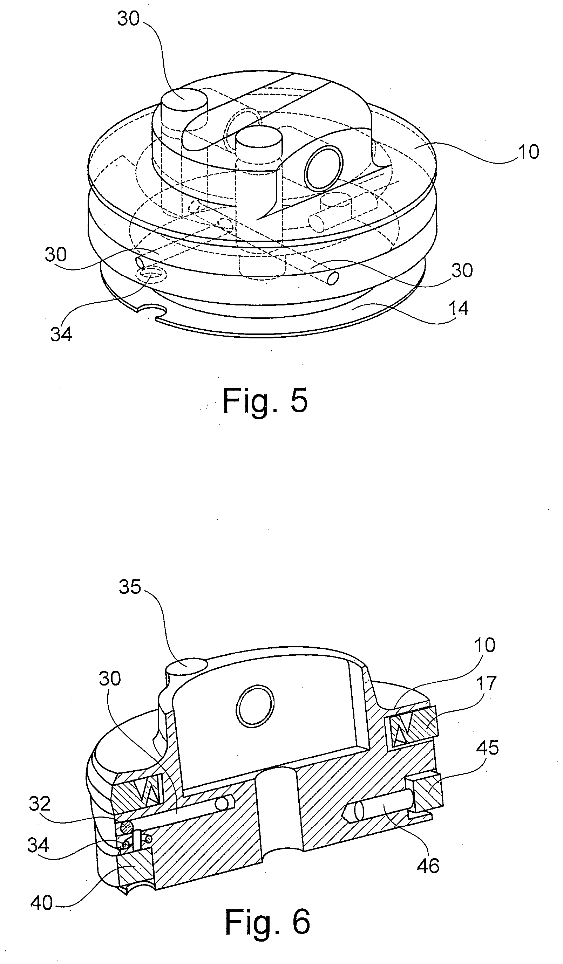

Figur 5 zeigt den Kolben 10 in einer teiltransparenten Darstellung ohne Dichtring 17 und ohne Verschlusselement 40. Die Kolbenringnut 14 ist ebenso zu erkennen wie die oberhalb davon angeordnete Ausnehmung für den Dichtring 17. Innerhalb der Kolbenringnut 14 ist in der unteren Seitenwand eine Bohrung zu erkennen, die aus fertigungstechnischen Gründen eingebracht ist, um die rechtwinklige Kanalführung der Auslasskanäle 30 und die Verbindung zu einer Öffnung in einer der Seitenwände der Kolbenringnut 14 zu erreichen. Die Auslasskanäle 30 sind dunkel dargestellt.

In der Figur 6 ist der Kolben 10 in einer Schnittdarstellung gezeigt, anhand der das Verschlusselement 40, die Dichtung 34 um die Mündungsbohrung in der einen Seitenwand der Kolbenringnut 14 zu erkennen ist. Der Stopfen 32 verschließt den Auslasskanal 30 nach radial außen, die innerhalb des Auslasskanals 30 ausgebildete Querbohrung führt zu dem Schalldämpfer 35 und entlässt die komprimierte Luft bei einer Richtungsumkehr des Kolbens 10 in die Umgebung. Ebenfalls ist das Spannelement 45 im Bereich der äußeren Wandung des Kolbens 10 zum radialen Aufweiten des Verschlusselementes 40 gezeigt. Dem Spannelement 45 kann ein Stellelement 46 zugeordnet sein, mit dem es möglich ist, das Spannelement 45 zu justieren. Alternativ oder zusätzlich dazu kann das Stellelement 46 auch als Positionierungshilfe verwendet werden, durch die erreicht wird, dass das Spannelement 45 gegen eine Drehung um die Zylinderlängsachse gesichert wird. Dabei kann das Stellelement 46 als separates Bauteil ausgebildet sein, das in der dafür vorgesehenen Vertiefung oder dem Schlitz angeordnet ist und in eine Ausnehmung oder Vertiefung am Spannelement 45 eingreift und so eine Drehung verhindert. Alternativ dazu kann das Stellelement 46 auch fest mit dem Spannelement 45 verbunden sein und in die Vertiefung, Bohrung oder Ausnehmung eingreifen, um die Drehung des Spannelementes 45 zu verhindern.

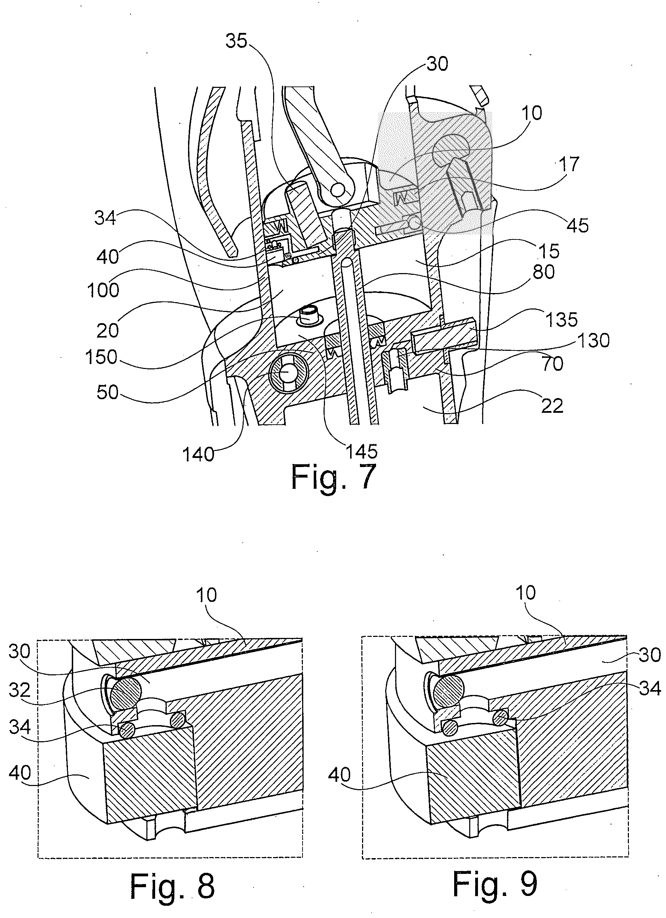

In der Figur 7 ist der Kolben gemäß den Figuren 3 bis 6 als oberer Abschluss einer Dämpfereinrichtung mit einem Gehäuse 100 dargestellt. Der Kolben 10 wird beispielsweise über eine Schubstange, die mit einer oberen Gelenkskomponente verbunden ist, entlang der Längserstreckung der Kolbenstange 80 in dem Zylinder des Gehäuses 100 bewegt. Wird der Kolben 10 zusammen mit der Kolbenstange 80 nach unten verlagert, also in Richtung auf den Träger 50, wird die Luft innerhalb der Flexionskammer 20 komprimiert. Aufgrund der Reibung des Verschlusselementes 40 an der Außenwand des Zylinders wird das Verschlusselement in Richtung auf die Dichtung 34 bewegt, die Auslasskanäle 30 werden geschlossen und keine Luft kann aus dem Schalldämpfer 35 in die Umgebung entweichen. Der Dichtring 17 bietet eine zusätzliche Abdichtung der Flexionskammer 20. Wird eine Umkehr-bewegung eingeleitet, also eine Extension, bewegt sich der Kolben 10 nach oben, das Verschlusselement 40 wird in Richtung auf die untere Kolbenringnutwand gedrückt und der Kanal 30 wird geöffnet. Luft kann aus der Flexionskammer 20 durch die Bohrungen innerhalb des Kolbens an der Dichtung 34 vorbei in die Umgebung hineinströmen. Die Extensionskammer weist ebenfalls einen Auslasskanal 130 und einen Schalldämpfer 135 auf, über die während der Extensionsbewegung Luft aus der Extensionskammer 22 in die Umgebung entlassen werden kann, ebenso ist es möglich, dass über ein Überströmventil 150 Luft aus der Umgebung oder aus der Extensionskammer 22 gesteuert in die Flexionskammer 20 gelangen kann, beispielsweise wenn bei einer Unterbrechung der Extensionsbewegung ein vollständiges Entlüften der Flexionskammer 20 erfolgt ist und darüber hinaus eine Extension stattfindet um zu verhindern, dass ein Unterdruck innerhalb der Flexionskammer 20 entsteht. Dafür ist ein separater Auslasskanal 140 zwischen der Flexionskammer 20 und der Extensionskammer 22 angeordnet, in dem ein Figur 7 nicht erkennbarer zusätzlicher Schalldämpfer 145 positioniert sein kann. Das Überströmventil 150 ist vorteilhafterweise ebenfalls als Rückschlagventil ausgebildet. Ebenso ist es vorgesehen, dass Luft aus der Flexionskammer 20 in die Extensionskammer 22 geleitet wird, wenn die Flexionskammern 20 entlüftet und gegebenenfalls die Extensionskammer 22 belüftet werden sollen.

Die Figur 8 zeigt in einer Detailansicht die Lage des Verschlusselementes 40 während der Flexionsbewegung. Es ist gut zu erkennen, dass das Verschlusselement 40 in Gestalt des Kolbenrings an der Dichtung 34 anliegt und den Auslasskanal 30 dichtend abschließt.

In der Figur 9 ist die Lage des Verschlusselementes 40 während einer Extensionsbewegung gezeigt, ein Spalt zwischen dem Verschlusselement 40 und der oberen Kolbenringnutwandung ist zu erkennen, wodurch die Durchlässigkeit von der Flexionskammer zum Auslasskanal 30 gewährleistet ist.

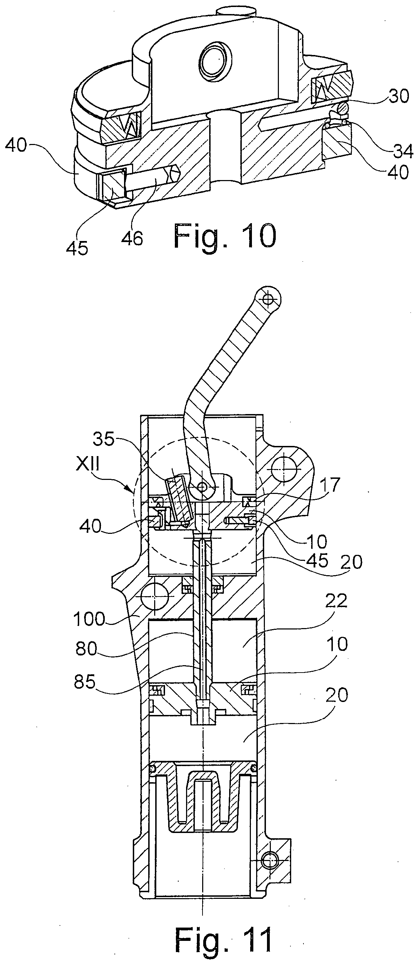

Figur 10 zeigt in einer Schnittdarstellung das Spannelement 45 mit dem Stellelement 46, das bewirkt, dass der Kolbenring 40 bzw. das Verschlusselement 40 gegen die Zylinderwandung gepresst wird. Über das Spannelement 45 und das Stellelement 46 kann die Reibungskraft zwischen dem Verschlusselement 40 und dem Kontaktbereich, also der Zylinderwand der Dämpfereinrichtung gesteuert werden. Je größer die Vorspannung und radiale Aufweitung des Verschlusselementes ist, desto größer wird die Reibung und desto exakter und präziser reagiert das Verschlusselement auf eine Richtungsumkehr. Neben der Reibungseinstellung wird über das Spannelement 45 gleichzeitig eine Verschleißkompensation erreicht.

Wie bereits dargelegt, kann das Stellelement 46 auch in einer nicht einstellbaren Ausgestaltung vorhanden sein. In diesem Fall dient es lediglich als Positionierelement, durch das eine Drehung des Spannelementes 45 um die Längsachse des Kolbens 80 herum verhindert wird.

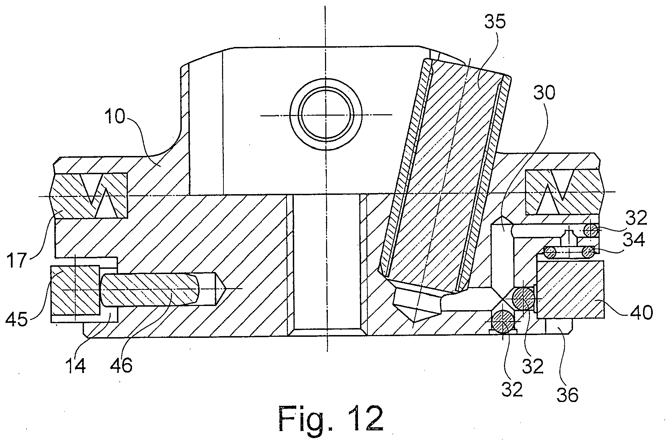

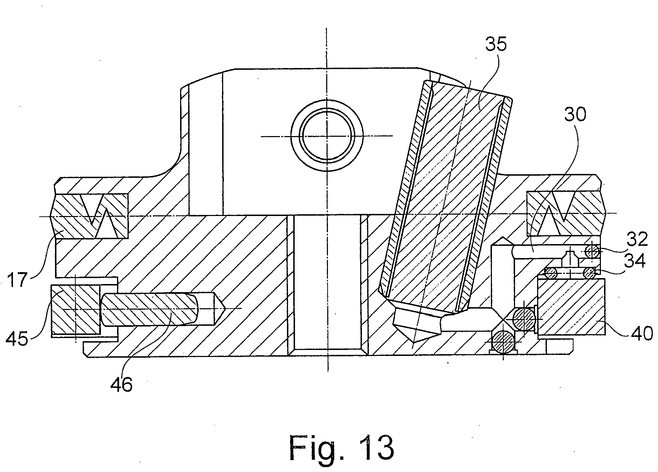

In der Figur 11 ist in einer Schnittdarstellung die komplette Dämpfereinrichtung gezeigt, in der zwei Flexionskammern 20 und eine Extensionskammer 22 angeordnet sind. Die beiden Flexionskammern 20 sind über die Kolbenstange 80 und den Verbindungskanal 85 strömungstechnisch miteinander gekoppelt. Der Aufbau des Kolbens 10, der die obere Flexionskammer 20 verschließt, ist im Detail in den Figuren 12 und 13 gezeigt und entspricht im Wesentlichen dem Aufbau gemäß der Figuren 3 bis 10. Bei einer Abwärtsbewegung des Kolbens 10 in Richtung auf die untere Kammerbegrenzung wird die Luft in beiden Flexionskammern 20 komprimiert, gleichzeitig wird der Auslasskanal 30 durch das Verschlusselement 40 verschlossen. Bei der Umkehrbewegung bleibt aufgrund der Reibung das Verschlusselement 40 an der Zylinderwand hängen, bis die untere Seitenwand der Kolbenringnut 14 das Verschlusselement 40 mit nach oben schiebt. Durch die Verlagerung innerhalb der Kolbenringnut 14 wird der Auslasskanal 30 geöffnet und komprimierte Luft kann an dem Verschlusselement vorbei durch den Auslasskanal 30 und dem Schalldämpfer 35 in die Umgebung entweichen. Ein unkontrolliertes Entweichen wird durch den oberen Dichtungsring 17 verhindert.

In der Figur 12 ist der Aufbau des Kolbens 10 in einer vergrößerten Schnittdarstellung gezeigt. Der Kolben 10 befindet sich in der Extensionsstellung, das heißt, dass das Verschlusselement 40 nicht an dem Dichtring 34 anliegt, so dass Luft aus der Flexionskammer 20 an dem Kolbenring 40 vorbei, beispielsweise im Bereich der Bohrung 36 für den Auslasskanal 30, in der oberen Kolbenringnutseitenwand in den Auslasskanal 30 hineinströmen kann. Von dem Auslasskanal 30 gelangt die Luft über den Schalldämpfer 35 in die Umgebung.

Figur 13 zeigt die Detaildarstellung gemäß Figur 12 in der Flexionsstellung, das Verschlusselement 40 liegt dichtend an dem Dichtring 34 an, der Auslasskanal 30 ist gegenüber der Flexionskammer 20 verschlossen und die Dämpfereinrichtung kann wie gewünscht die Luft in der Flexionskammer komprimieren.

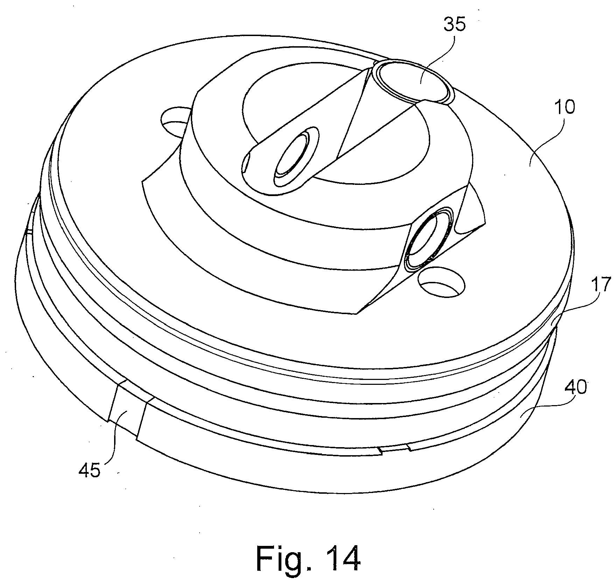

Figur 14 zeigt eine perspektivische vergrößerte Darstellung des Kolbens 10 mit dem Dichtring 17 mit dem Verschlusselement 40 und dem aufweitenden Spannelement 45.

In den bisherigen Ausführungsbeispielen wurde stets die obere Flexionskammer 20 entlüftet. Natürlich ist es auch denkbar, zusätzlich oder alternativ dazu die untere Flexionskammer 20 zu entlüften. In diesem Fall wird der Auslasskanal 30 als zweite Bohrung durch die Kolbenstange 80 geführt, so dass auf diese Weise eine Entlüftung in die Atmosphäre ermöglicht wird. Natürlich kann auf analoge Weise auch eine Extensionskammer entlüftet werden.

Durch das Spannelement 45 kann auch insbesondere das Verschlusselement 40 besser und zuverlässiger positioniert werden. Eine konkrete Ausführungsform sieht vor, ein Spannelement 45 mit ovalem Querschnitt in eine dafür vorgesehene ovale Aussparung im Bereich des Kolbenringschlitzes einzusetzen. Auf diese Weise wird nicht nur eine Drehung, sondern auch eine Torsion des Kolbenrings verhindert.

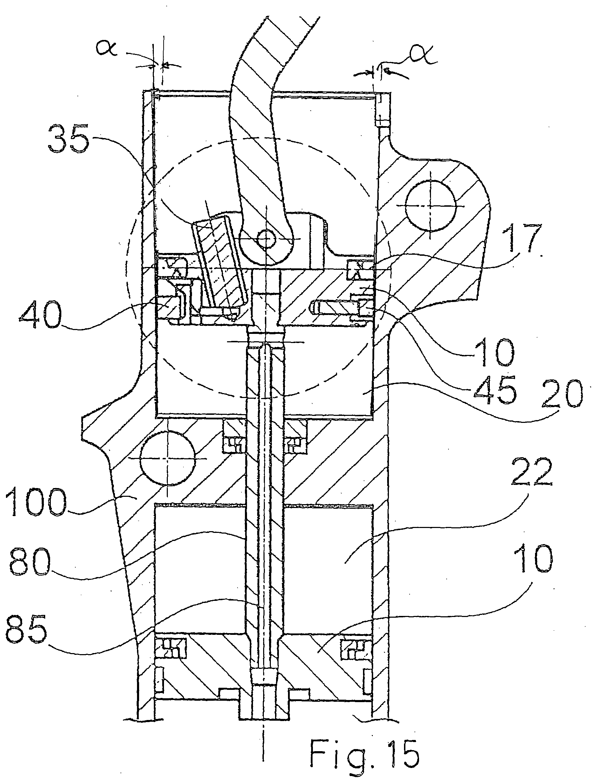

In der Figur 15 ist eine Variante der Figur 11 dargestellt, anhand der exemplarisch gezeigt ist, dass die Kammer 20 bzw. der Zylinder, in dem der Kolben 10 längsverschieblich verfahren wird, eine konische Aufweitung im dargestellten Ausführungsbeispiel nach oben aufweist, so dass bei einer Flexionsbewegung eines Prothesenkniegelenkes der stets vorhandene Spalt zwischen dem Kolben 10 und der Kammerwandung zunehmend kleiner wird. Im dargestellten Ausführungsbeispiel beträgt der Konuswinkel α ca. 1°, die Konizität kann grundsätzlich davon abweichen. Der Konuswinkel α ist so bemessen, dass sowohl an dem oberen als auch dem unteren Umkehrpunkt der Kolbenbewegung einerseits eine Verschieblichkeit innerhalb der Kammer 20 gegeben und andererseits eine ausreichende Abdichtung über den Dichtungsring gewährleistet ist. Das Verschlusselement 40 gleitet an der Innenseite der Kammer 20 entlang und wird durch das radial wirkende Spannelement 45 gegen die Kammerwandung gedrückt. Bei zunehmender Flexion und einer Bewegung des Kolbens 10 nach unten erhöht sich der Druck des Verschlusselementes 40 auf die Innenwand aufgrund des sich verringernden Spaltes zwischen dem Kolben 10 und der Kammerinnenwand, was dazu führt, dass eine erhöhte Friktion und damit eine verbesserte Schaltbarkeit des Verschlusselementes in dem Endbereich des Hubes erfolgt. Sobald eine Bewegungsumkehr stattfindet, bleibt aufgrund der höheren Anpresskraft das Verschlusselement 40 besser an der Kammerwand haften, so dass sich der Auslasskanal 30 zuverlässig öffnet. Neben der dargestellten gleichmäßigen konischen Verjüngung in Flexionsrichtung, also in Richtung auf den unteren Umkehrpunkt, ist es auch möglich, durch eine stufenweise konische Ausgestaltung unterschiedliche Anpresskraftniveaus zu erzeugen. So ist es möglich, im oberen Bereich, also im Bereich der maximalen Extension, eine zylindrische oder nahezu zylindrische Ausgestaltung der Kammer 20 vorzusehen, während ungefähr ab der Mitte der Hubbewegung eine zunehmende Verjüngung oder eine Vergrößerung des Konuswinkels α vorgesehen ist, um einerseits eine leichte Beweglichkeit bei einer geringen Beugung und andererseits eine verbesserte Schaltzuverlässigkeit bei einer hohen Beugung oder maximalen Flexion zu gewährleisten. Bei einer Variante gemäß der Figur 1 ist die Kolbenstange 80 mit unterschiedlichen Durchmessern versehen, wobei die Kolbenstange 80 einen sich nach oben hin vergrößernden Querschnitt aufweist, bevorzugt eine konische Erweiterung, so dass das Schaltelement 75 bei einer zunehmenden Flexion des Gelenkes und einer Verlagerung des Kolbens 10 nach unten mit einer sich erhöhenden Spannung von außen an der Kolbenstange 80 anliegt. Die konische Aufweitung der Kolbenstange 80 kann nur bereichsweise, vorzugsweise in dem Bereich der Endlage der Kolben 10, ausgebildet sein, ebenso muss die Aufweitung nicht mit einer konstanten Steigung ausgeführt sein, die Konizität kann sich über den Verlagerungsbereich verändern.

In der Figur 15 ist zu erkennen, dass die Kolbenstange 80 durch das Sperrelement, dass die beiden Kammern 20 voneinander trennt hindurch geht. Die unter Kammer 20 steht mit der obere Kammer 20 über eine Längsbohrung und eine Querbohrung in der Kolbenstange 80 in strömungstechnischer Verbindung, so dass eine Entlüftung der oberen Kammer 20 in die zweite Kammer 22 oder die freie Umgebung gleichzeitig auch eine Entlüftung der unteren Kammer 20 bewirkt. Durch die Entlüftung bei einer Entlastung wird der Rückfedereffekt am Ender der Flexionsphase verhindert.

Durch die Öffnung des Auslasskanals bei einer Bewegungsumkehr ist es möglich, den Überdruck in der ersten Kammer 20 in die Umgebung bzw. in eine zweite Kammer 22 zu transferieren, so dass die bei der Flexion komprimierte Luft in die Umgebungsatmosphäre entlassen wird, so dass ein vollständiger Druckausgleich stattfindet.

## Patentansprüche

1. Orthopädietechnische Dämpfereinrichtung mit einem beweglich gelagerten Kolben (10) und zumindest einer eine Wandung (15) aufweisende Kammer (20), in der ein komprimierbares Medium durch Verlagerung des Kolbens (10) in einer ersten Richtung verdichtet und durch Verlagerung des Kolbens (10) in eine entgegengesetzte zweite Richtung entspannt wird, wobei die Kammer (20) mit einem Auslasskanal (30) verbunden ist, dem ein Verschlusselement (40) zugeordnet ist, **dadurch gekennzeichnet, dass**
a. das Verschlusselement (40) beweglich an einem Träger (50) gelagert,
b. der Träger (50) mit dem Kolben (10) gekoppelt oder als Kolben (10) ausgebildet und
c. das Verschlusselement (40) mit einem relativ zu dem Träger (50) verlagerbaren Kontaktbereich (60) gekoppelt ist.

2. Dämpfereinrichtung nach Anspruch 1, **dadurch gekennzeichnet, dass** der Kontaktbereich (60) die Wandung (15) der Kammer (20) oder eines Sperrelementes (50) ist.

3. Dämpfereinrichtung nach Anspruch 1 oder 2, **dadurch gekennzeichnet, dass** der Kolben (10) als Drehkolben oder zylindrischer Kolben ausgebildet ist.

4. Dämpfereinrichtung nach einem der voranstehenden Ansprüche, **dadurch gekennzeichnet, dass** der Auslasskanal (30) in die Umgebungsatmosphäre oder eine zweite Kammer (22) mündet.

5. Dämpfereinrichtung nach einem der voranstehenden Ansprüche, **dadurch gekennzeichnet, dass** in oder an dem Auslasskanal (30) ein Schalldämpfer (35) angeordnet ist.

6. Dämpfereinrichtung nach einem der voranstehenden Ansprüche, **dadurch gekennzeichnet, dass** das Verschlusselement (40) reibend und gleitend an dem Kontaktbereich (60) gelagert ist.

7. Dämpfereinrichtung nach einem der voranstehenden Ansprüche, **dadurch gekennzeichnet, dass** das Verschlusselement (40) gegen den Kontaktbereich (60) elastisch vorgespannt ist.

8. Dämpfereinrichtung nach einem der voranstehenden Ansprüche, **dadurch gekennzeichnet, dass** der Auslasskanal (30) durch den Kolben (10) geführt ist.

9. Dämpfereinrichtung nach einem der voranstehenden Ansprüche, **dadurch gekennzeichnet, dass** das Verschlusselement (40) als Kolbenring ausgebildet und in einer Kolbenringnut (14) gelagert ist.

10. Dämpfereinrichtung nach einem der Ansprüche 1 bis 8, **dadurch gekennzeichnet, dass** das Verschlusselement (40) an einer Kolbenstange (80) und in einem die Kolbenstange (80) umgebenden Sperrelement (70) gelagert ist.

11. Dämpfereinrichtung nach einem der voranstehenden Ansprüche, **dadurch gekennzeichnet, dass** das Verschlusselement (40) im verschließenden Zustand an einer den Auslasskanal (30) umgebenden Dichtung (34) anliegt.

12. Dämpfereinrichtung nach einem der voranstehenden Ansprüche, **dadurch gekennzeichnet, dass** eine zweite Kammer (22) auf der der ersten Kammer (20) gegenüberliegenden Seite des Kolbens (10) angeordnet ist, in der das komprimierbare Medium verdichtet wird.

13. Dämpfereinrichtung nach einem der Ansprüche 2 bis 12, **dadurch gekennzeichnet, dass** das Sperrelement (70) in einem die Kammern (20, 22) ausbildenden Gehäuse (100) ortsfest gelagert ist.

14. Dämpfereinrichtung nach einem der voranstehenden Ansprüche, **dadurch gekennzeichnet, dass** das Verschlusselement (40) über ein Schaltelement (75) mit dem Kolben (10) gekoppelt ist.

15. Dämpfereinrichtung nach einem der voranstehenden Ansprüche, **dadurch gekennzeichnet, dass** das Verschlusselement (40) und/oder der Auslasskanal (30) einstellbar ausgebildet sind.

16. Dämpfereinrichtung nach einem der voranstehenden Ansprüche, **dadurch gekennzeichnet, dass** die Kammer (20) in Richtung auf eine Endstellung des Kolbens (10) sich konisch verjüngend ausgebildet ist.

17. Dämpfereinrichtung nach einem der voranstehenden Ansprüche, **dadurch gekennzeichnet, dass** der Träger (50) ortsfest innerhalb der Kammer (20) angeordnet ist.

18. Dämpfereinrichtung nach Anspruch 4, **dadurch gekennzeichnet, dass** die zweite Kammer (22) als offene Kammer ausgebildet ist und einen gegenüber der ersten Kammer (20) geringeren Innendruck aufweist.

## Claims

1. Orthopedic damping device with a movably mounted piston (10) and at least one chamber (20) which has a wall (15) and in which a compressible medium is compressed by moving the piston (10) in a first direction and decompressed by moving the piston (10) in an opposite, second direction, wherein the chamber (20) is connected to an outlet channel (30), which is assigned a closure element (40), **characterized in that**
a. the closure element (40) is movably mounted on a support (50),
b. the support (50) is coupled to the piston (10) or is formed as a piston (10), and
c. the closure element (40) is coupled to a contact region (60) which can be moved relative to the support (50).

2. Damping device as claimed in claim 1, **characterized in that** the contact region (60) is the wall (15) of the chamber (20) or of a blocking element (50).

3. Damping device as claimed in claim 1 or 2, **characterized in that** the piston (10) is formed as a rotary piston or cylindrical piston.

4. Damping device as claimed in one of the preceding claims, **characterized in that** the outlet channel (30) leads into the surrounding atmosphere or a second chamber (22).

5. Damping device as claimed in one of the preceding claims, **characterized in that** a sound damper (35) is arranged in or on the outlet channel (30).

6. Damping device as claimed in one of the preceding claims, **characterized in that** the closure element (40) is mounted frictionally and slidingly on the contact region (60).

7. Damping device as claimed in one of the preceding claims, **characterized in that** the closure element (40) is resiliently pre-loaded against the contact region (60).

8. Damping device as claimed in one of the preceding claims, **characterized in that** the outlet channel (30) is guided through the piston (10).

9. Damping device as claimed in one of the preceding claims, **characterized in that** the closure element (40) is formed as a piston ring and is mounted in a piston ring groove (14).

10. Damping device as claimed in one of claims 1 to 8, **characterized in that** the closure element (40) is mounted on a piston rod (80) and in a blocking element (70) surrounding the piston rod (80).

11. Damping device as claimed in one of the preceding claims, **characterized in that** the closure element (40) in the closing state bears against a seal (34) surrounding the outlet channel (30).

12. Damping device as claimed in one of the preceding claims, **characterized in that** a second chamber (22) is arranged on the side of the piston (10) opposite the first chamber (20), in which second chamber the compressible medium is compressed.

13. Damping device as claimed in one of claims 2 to 12, **characterized in that** the blocking element (70) is mounted in a stationary manner in a housing (100) forming the chambers (20, 22).

14. Damping device as claimed in one of the preceding claims, **characterized in that** the closure element (40) is coupled to the piston (10) via a switching element (75).

15. Damping device as claimed in one of the preceding claims, **characterized in that** the closure element (40) and/or the outlet channel (30) is/are adjustable.

16. Damping device as claimed in one of the preceding claims, **characterized in that** the chamber (20) tapers conically in the direction of an end position of the piston (10).

17. Damping device as claimed in one of the preceding claims, **characterized in that** the support (50) is arranged in a stationary manner inside the chamber (20).

18. Damping device as claimed in claim 4, **characterized in that** the second chamber (22) is formed as an open chamber and has a lower internal pressure compared with the first chamber (20).

## Revendications

1. Dispositif d'amortissement orthopédique comportant un piston monté mobile (10) et au moins une chambre (20) comprenant une paroi (15), chambre dans laquelle un fluide compressible est comprimé par déplacement du piston (10) dans une première direction et est détendu par déplacement du piston (10) dans une seconde direction opposée, la chambre (20) étant reliée à un canal de sortie (30) auquel est associé un élément d'obturation (40), **caractérisé en ce que**
a. l'élément d'obturation (40) est monté mobile sur un support (50),
b. le support (50) est couplé au piston (10) ou est réalisé sous forme de piston (10), et
c. l'élément d'obturation (40) est couplé à une zone de contact (60) mobile par rapport au support (50).

2. Dispositif d'amortissement selon la revendication 1,
**caractérisé en ce que**
la zone de contact (60) est la paroi (15) de la chambre (20) ou d'un élément de blocage (50).

3. Dispositif d'amortissement selon la revendication 1 ou 2,
**caractérisé en ce que**
le piston (10) est réalisé sous forme de piston rotatif ou de piston cylindrique.

4. Dispositif d'amortissement selon l'une des revendications précédentes,
**caractérisé en ce que**
le canal de sortie (30) débouche dans l'atmosphère ambiante ou dans une seconde chambre (22).

5. Dispositif d'amortissement selon l'une des revendications précédentes,
**caractérisé en ce que**
un amortisseur de bruit (35) est agencé dans ou sur le canal de sortie (30).

6. Dispositif d'amortissement selon l'une des revendications précédentes,
**caractérisé en ce que**
l'élément d'obturation (40) est monté en friction et en coulissement sur la zone de contact (60).

7. Dispositif d'amortissement selon l'une des revendications précédentes,
**caractérisé en ce que**
l'élément d'obturation (40) est élastiquement précontraint contre la zone de contact (60).

8. Dispositif d'amortissement selon l'une des revendications précédentes,
**caractérisé en ce que**
le canal de sortie (30) est mené à travers le piston (10).

9. Dispositif d'amortissement selon l'une des revendications précédentes,
**caractérisé en ce que**
l'élément d'obturation (40) est réalisé sous forme de segment de piston et est monté dans une gorge à segment de piston (14).

10. Dispositif d'amortissement selon l'une des revendications 1 à 8,
**caractérisé en ce que**
l'élément d'obturation (40) est monté sur une tige de piston (80) et dans un élément de blocage (70) entourant la tige de piston (80).

11. Dispositif d'amortissement selon l'une des revendications précédentes,
**caractérisé en ce que**
dans l'état d'obturation, l'élément d'obturation (40) prend appui contre un joint d'étanchéité (34) entourant le canal de sortie (30).

12. Dispositif d'amortissement selon l'une des revendications précédentes,
**caractérisé en ce que**
une seconde chambre (22) est agencée sur le côté du piston (10) opposé à la première chambre (20), dans laquelle le fluide compressible est comprimé.

13. Dispositif d'amortissement selon l'une des revendications 2 à 12,
**caractérisé en ce que**
l'élément d'obturation (40) est monté de façon stationnaire dans un boîtier (100) réalisant les chambres (20, 22).

14. Dispositif d'amortissement selon l'une des revendications précédentes,
**caractérisé en ce que**
l'élément d'obturation (40) est couplé au piston (10) par un élément de commutation (75).

15. Dispositif d'amortissement selon l'une des revendications précédentes,
**caractérisé en ce que**
l'élément d'obturation (40) et/ou le canal de sortie (30) sont réalisés de façon réglable.

16. Dispositif d'amortissement selon l'une des revendications précédentes,
**caractérisé en ce que**
la chambre (20) est réalisée de manière à se rétrécir coniquement en direction d'une position finale du piston (10).

17. Dispositif d'amortissement selon l'une des revendications précédentes,
**caractérisé en ce que**
le support (50) est agencé de façon stationnaire à l'intérieur de la chambre (20).

18. Dispositif d'amortissement selon la revendication 4,
**caractérisé en ce que**
la seconde chambre (22) est réalisée sous forme de chambre ouverte et présente une pression intérieure inférieure à celle de la première chambre (20).
